# EUROPEAN PATENT SPECIFICATION

(11) **EP 0 597 902 B1**
(45) Date of publication and mention of the grant of the patent: **05.03.1997**
(21) Application number: 92915863.2
(22) Date of filing: 23.07.1992
(51) Int. Cl.: G01N 33/53, G01N 33/563, G01N 33/573, G01N 33/74, G01N 33/76

(54) **METHOD FOR ELIMINATING ERRONEOUS RESULTS IN AN IMMUNOASSAY**
VERFAHREN ZUR BESEITIGUNG VON FALSCHEN ERGEBNISSEN IN EINEM IMMUNOASSAY
PROCEDE D'ELIMINATION DE RESULTATS ERRONES DANS UN DOSAGE IMMUNOLOGIQUE

(30) Priority: 26.07.1991 US 736156
(43) Date of publication of application: 25.05.1994
(73) Proprietor: Dade Chemistry Systems Inc., Deerfield, Illinois 60015-0778 (US)
(72) Inventor: VAIDYA, Hemant, Chunilal, Wilmington, DE 19808 (US)
(74) Representative: von Kreisler, Alek, Dipl.-Chem.
(86) International application number: US9206006
(87) International publication number: WO9303366

(56) References cited:
- EP-A- 0 331 068
- EP-A- 0 440 044
- EP-A- 0 452 503
- WO-A-90/10226
- WO-A-91/06559
- WO-A-91/06857
- WO-A-91/16627
- US-A- 4 912 033

## Description

### Field of the Invention

This invention relates to a method for eliminating erroneous results in an immunoassay and, in particular, to eliminating erroneous results due to the presence of interfering antibodies which are capable of interacting with the capture antibody and/or detector antibody by using an immunoreactive antibody fragment conjugate as the detector antibody and non-polymerized IgG.

### Background of the Invention

Immunoassays are used as a diagnostic tool to screen for the presence or absence of an antigen in a biological sample such as serum, plasma, blood or the like. Typically, such assays involve the use of two or more antibodies directed against different antigenic determinants which can be reacted in a variety of formats. For example, in a forward sandwich assay format a first antibody, which is either in solution or immobilized to a solid phase, is incubated with the sample suspected to contain the antigen. If antigen is present, then a first antibody-antigen complex will form and the resulting complex is then reacted with a second antibody conjugated to a reporter to produce a first antibody-antigen-second antibody-reporter complex which can then be detected.

Unfortunately, immunoassays can malfunction to produce an erroneous result even though no antigen is present in the sample. Erroneous results can arise in a number of ways such as when the second-antibody-reporter conjugate reacts with the first antibody despite the fact that no antigen is present in the sample. This can occur due to non-specific interactions such as hydrophobic interactions, ionic interactions, hydrogen bonds, Van der Waals forces, and the like.

The occurrence of nonspecific interactions can also be attributed to substances present in the sample to be tested which bind to both the first antibody and/or second antibody conjugate. Such substances are usually present in serum samples from animals previously exposed to the immunoglobulins of the species used to produce the first and/or second antibodies. These immunogiobulins act as anantigen which causes the formation of interfering antibodies in that animal.

Methods to prevent erroneous results include using homopolymers of IgG or Fab or F(ab')₂ fragments thereof or heteropolymers of an IgG molecule and and Fc-free IgG fragment originating from the same species of animal as one of the specific immunoreactants as is described in U.S. Patent No. 4,914,040, issued to Lenz et al. on April 3, 1990.

WO 90/10226 published on September 7, 1990 describes preventing false positive results in ELISA-assay methods by using a small quantity of interference antibody complexing serum (IACS) from the species used to prepare the capture and indicator antibodies (intact antibody coupled to an enzyme) with the interference antibody in the sample to be tested for antigen. The IACS polyclonal antibodies react with the interference antibody to prevent the interference antibody from linking the capture antibody and the indicator antibody.

### Summary of the Invention

This invention concerns a method for substantially eliminating erroneous results in an immunoassay due to the presence of interfering antibodies which comprises reacting an immunoreactive antibody fragment conjugate as the detector antibody and non-polymerized IgG with the interfering antibodies present in a sample to be tested for the presence or absence of an analyte.

### Brief Description of the Drawings

Figure 1 depicts CKMB values obtained in an immunoassay format in which an intact antibody conjugate was used as the detector antibody in the presence or absence of polymerized IgG. The results show that CKMB values in 18 samples were greater than 12 ng/ml and could not be reduced further with 24 µg of polymerized IgG.

Figure 2 shows that CKMB values of 3 of the 18 samples described in Figure 1 above having greatly elevated CKMB values could not be reduced below 12 ng/mL using as much as 80 µg polymerized IgG per assay.

Figure 3 shows that non-polymerized polyclonal IgG could not substantially reduce non-specific binding when detector antibody was an intact antibody conjugate.

Figure 4 shows that substantially all non-specific binding was eliminated using an F(ab')₂ conjugate as the detector antibody and non-polymerized polyclonal IgG.

Figure 5 shows that substantially all non-specific binding was eliminated using an F(ab')₂ conjugate as the detector antibody in the presence or absence of non-polymerized polyclonal IgG. CKMB values using an intact antibody conjugate as the detector antibody in the absence and presence of polymerized IgG and non-polymerized IgG were compared to the values obtained using an F(ab')₂ conjugate as the detector antibody in the absence and presence of polymerized IgG and non-polymerized IgG.

Figure 6 shows the hCG measurements determined for each of the three patient samples in the presence and absence of non-polymerized mouse IgG.

### Detailed Description of the Invention

The term "capture antibody" as used herein means an antibody whether polyclonal, monoclonal, or an immunoreactive fragment thereof which is capable of binding analyte. It can be used in either a heterogeneous (solid phase) or homogeneous (solution phase) assay.

The term "detector antibody" as used herein means an antibody conjugated to a reporter whether the antibody is polyclonal, monoclonal, or an immunoreactive fragment thereof which is capable of binding analyte at an epitope different from that bound by the capture antibody.

The term "immunoreactive antibody fragment" as used herein means a fragment or fragments which contain the binding region of the antibody. Such fragments can be Fab-type fragments which are defined as fragments devoid of the Fc portion, e.g., Fab, Fab', and F(ab')₂ fragments.

The term "interfering antibody" as used herein means an antibody present in the sample which is capable of interacting with the capture antibody and/or the immunoreactive fragment of the detector antibody.

The term "substantially eliminate" as used herein means that an imperceptible amount of non-specific binding may occur, i.e., an amount which does not produce an erroneous result.

According to the present invention, a method is provided for substantially eliminating erroneous results in an immunoassay due to the presence of interfering antibodies which comprises reacting an immunoreactive antibody fragment conjugate as the detector antibody and non-polymerized IgG with the interfering antibodies present in a sample to be tested for the presence or absence of an analyte.

The immunoreactive antibody fragment conjugate and non-polymerized IgG can be reacted with the interfering antibodies in the biological sample at different steps in the immunoassay process. They can be added simultaneously to the sample after it has been incubated with the capture antibody. The capture antibody, immunoreactive antibody fragment conjugate and non-polymerized IgG can be reacted simultaneously with the sample suspected to contain interfering antibodies. The immunoreactive antibody fragment conjugate and non-polymerized IgG can react with the sample before incubating with the capture antibody. In another variation, sample containing interfering antibodies can be allowed to react with non-polymerized IgG before incubating with capture antibody and immunoreactive antibody fragment conjugate. Preferably, the immunoreactive antibody fragment conjugate and non-polymerized IgG are reacted simultaneously with the sample containing analyte and the capture antibody.

The amount of non-polymerized IgG used should be an amount sufficient to complex substantially all the interfering antibodies present in the sample. The amount will vary depending upon the sample size, when the reagents are added.

The method of the invention can be used in virtually any immunoassay format be it ona solid phase or in solution. Such assays include but are not limited to forward sandwich assays, competitive assays, reverse sandwich assays as described in U.S. Patent No. 4,098,876,
and simultaneous assays as described in U.S. Patent No. 4,244,940.

Immunometric assays using monoclonal antibodies are described in U.S. Patent Nos. 4,376,110 and 4,486,530.

If a solid phase immunoassay is employed, any one of a wide variety of supports is available. There can be mentioned synthetic polymer supports such as polystyrene, polypropylene, substituted polystyrene, e.g., aminated or carboxylated polystyrene, polyacrylamides, polyamides and polyvinylchloride ; glass beads and agarose. Preferably, the solid phase is a coated chromium dioxide particle as described in U.S. Patent No. 4,661,408, issued to Lau et al. on April 28, 1987.

These chromium dioxide particles are sufficiently hydrolytically stable to be useful as solid supports in heterogeneous immunoassays and bioaffinity separations. The core of the particles is acicular, rutile chromium dioxide having a surface area of 5-100 m²/g, coercivity of 100-750 oersteds, remanent magnetization of 5-45 emu/g and saturation magnetization of 8-85 emu/g. These particles are surface stabilized and further stabilized with a coating of SiO₂. The silica coated chromium dioxide is then further coated with a silane to both further stabilize the particle and to provide binding sites for proteins.

The capture antibody can be polyclonal, monoclonal, or an immunoreactive fragment thereof as described above and can be prepared using techniques well known to those skilled in the art.

The capture antibody, immunoreactive antibody fragment and non-polymerized IgG can be prepared from practically any species capable of producing antibodies such as a mouse, rat, rabbit, goat, or horse. In the preferred embodiment of this invention, antibodies and immunoreactive fragments are prepared from murine cells.

Non-polymerized IgG can be prepared using standard techniques for producing antibodies be they monoclonal or polyclonal. It is preferred in practicing the invention that the non-polymerized IgG used be derived from a non-immune source. It is further preferred that the non-polymerized IgG be from the same species as the capture antibody and the conjugate.

The immunoreactive antibody fragment conjugate is an immunoreactive antibody fragment coupled to a reporter using conventional coupling and labeling techniques. A wide variety of reporters are available for coupling to the immunoreactive antibody fragment. Reporters can be a radioactive isotope such as ¹²⁵I, enzymes, fluorogenic, chemiluminescent, or electrochemical materials.

Examples of reporter enzymes which can be used to practice the invention include hydrolases, lyases, oxidoreductases, transferases, isomerases, and ligases. Specific examples include alkaline phosphatase, betagalactosidase and horseradish peroxidase. Other examples can be found in Ishikawa et al., Clin. Chem. Acta, 194:51-74 (1990).

The invention is suitable for determining virtually any analyte. There can be mentioned the MB isoenzyme of creatine kinase (CKMB), thyroid stimulating hormone (TSH), lutenizing hormone (LH), follicle stimulating hormone (FSH), prolactin, ferritin, alpha-fetoprotein (AFP) and human chorionic gonadotropin (hCG).

The following examples are intended to illustrate the practice of the invention.

### Examples

All examples discussed below utilized an aca® discrete clinical analyzer (E. I. du Pont de Nemours and Company, Wilmington, DE 19898). An example of an aca® is described in U.S. Patent No. 4,066,412.

In addition, as was noted above, the method of the invention can be practiced employing any antibodies and/or immunoreactive fragments thereof suitable for use in an immunoassay format.

The following reagents and protocols were used in the examples described below:

### a) Cell lines used in the CKMB immunoassay

The cell lines producing the monoclonal antibodies employed were obtained using the procedure described in U.S. Patent No. 4,912,033 and Vaidya et al., Clin. Chem., 32(4):857-663 (1986).

Once ascites production is complete, monoclonal antibodies so obtained can be purified using any number of standard techniques such as ammonium sulfate precipitation dialysis, affinity chromatography and ion exchange chromatography. These and other methods for the isolation and purification of monoclonal antibodies are described in general by Goding, Monoclonal Antibodies: Principles and Practice, Academic press, London and New York, 1983 and U.S. Patent 4,533,496.

The preferred method for purification and isolation was affinity chromatography on protein A sepharose (Pharmacia Fine Chemicals, Uppsala, Sweden). Protein A is a polypeptide (MW 42,000) isolated from Staphylococcus aureus which binds immunoglobulin without interacting with the antigen binding site.

Anti-CKB monoclonal antibody used as the capture reagent immobilized on chromium dioxide particles as described below was obtained as described above. The clone number was 2581 BH 1.1, and the monoclonal antibody was IgG₁ subclass. As was noted above, this invention is not limited to use of a particular antibody and/or immunoreactive fragment. For example, any anti-CKMB antibody useful in an immunoassay can be used.

The anti-CKMB monoclonal antibody used to produced the immunoreactive fragment described below was obtained as described above. The clone number was 2580 CC 4.2 and the monoclonal antibody was an IgG_{2b} subclass.

Protein A purified antibody was dialyzed overnight at 4°C against acetate buffer (100 mM sodium acetate, 150 mM sodium chloride, pH 3.5). The dialyzed antibody was diluted to a concentration of 5 mg/ml using dialysis buffer. The antibody solution was placed in a waterbath at 37°C for 5 to 10 minutes.

A 10 mg/ml solution of pepsin (Sigma Chemical Co., St. Louis, MO) was prepared in acetate buffer. The amount of pepsin required to give a weight ratio of antibody to pepsin of 50:1 was calculated. The antibody solution was stirred while adding the pepsin. The mixture was incubated for 10-15 minutes. The reaction was stopped by slowly adding 3.5 M Tris base dropwise until the pH of the solution was in the range of 7.0 to 8.0. The F(ab')₂ preparation was passed through 15-20 mL of protein A - sepharose in a 2.2 x 25 cm column at a flow rate of 4-4.5 ml per hour. The protein peak was monitored by recording the absorbance of the fractions at 280 nm. The protein peak was collected and concentrated to about 30 mg/ml using an Amicon stirred cell fitted with 62 mm PM 30 membrane filter. Sterilized F(ab')₂ concentrate was filtered and stored at -20°C.

### b) Preparation of F(ab')₂ β-galactosidase conjugate

Anti-CKMB antibody fragment as described above was coupled to β-galactosidase using the procedure described by Kitagawa et al., Enzyme labeling with N-hydroxysuccinimidyl ester of maleimide in "Enzyme Immunoassays", Ishikawa et al., Eds., pp 81-90 (1981).

The conjugate was prepared as follows:
Anti-CKMB monoclonal antibody F(ab')₂ fragment was dialyzed against antibody dialysis buffer, (20 mM phosphate buffer, 300 mM NaCl, pH 7.0). One mole of F(ab')₂ was mixed with 30 moles of SMCC [N-succinimidyl 4-(N-maleimido methyl) cyclohexane-1-carboxylate] and incubated at room temperature for 35 min with constant stirring.. The mixture was loaded on a Sephadex® G-25 column (2.2 X 13 cm) equipped with the UV detector (absorbance 280 nm). Activated F(ab')₂ fragment was eluted using antibody dialysis buffer. The protein peak was collected. Volume was recorded and the protein concentration was estimated. One mole of E. coli β-galactosidase [equivalent to SMCC activated F(ab')₂] purchased from Boehringer Mannheim was dissolved in antibody dialysis buffer. Activated F(ab')₂ was mixed with β-galactosidase and incubated for at least 25 min at 25°C constantly stirring. Synthesis of the conjugate was monitored using a LKB HPLC equipped with 100 µl loop GF 450 analytical column. The reaction was quenched when the leading peak extended beyond the second peak on the chromatogram by adding 10 µl of 0.1 M N-ethylmalemide solution for every ml of conjugate reaction mixture. The mixture was concentrated to 4.0 mL using an Amicon stir cell and YM 100 filter (both from Amicon). Conjugate concentrate was filtered through 0.2 µm syringe filter and purified using LKB HPLC equipped with 1 ml loop GF 450 column, UV monitor, fraction collector and chart recorder. Appropriate fractions were collected and pooled and absorbance was measured at 280 nm wavelength to estimate the protein concentration. The resulting concentrate was filtered, sterilized and stored at 4°C. Conjugate concentrate was diluted as needed in the β-galactosidase conjugate dilution buffer (33.5 g PIPES [piperazine-N,N'-bis[2-ethanesulfonic acid]) 0.2 g MgCl₂, 29.2 g NaCl, 100 g bovine serum albumin, and 0.167 g mouse IgG per liter of deionized water, pH 7.0) for the CKMB assay.

### c) Anti-CKB Capture Antibody Coupled to Chromium Dioxide Particles

Anti-CKB antibody was immobilized on chromium dioxide particles according to the procedure substantially as described by Birkmeyer et al. (Birkmeyer, et al., Application of novel chromium dioxide magnetic particles to immunoassay development, Clin. Chem., 33:1543-1547, 1987).

4 liters of coupling buffer was prepared by mixing 196 ml of 10 mM KH₂PO₄ monobasic and 204 ml of 10 mM K₂HPO₄ dibasic and increasing the volume to 4 liters using deionized water. pH was adjusted to 7.0. Anti-CKB monoclonal antibody (2581 BH 1.1) was dialyzed overnight against coupling buffer which was changed and dialysis continued for an additional four hours. The volume of the dialyzed antibody was measured, and protein concentration was estimated. The antibody solution was diluted to 2 mg/ml with the coupling buffer. Equal volumes of diluted antibody (2 mg/ml) and 5% suspension of gluteraldehyde activated chromium dioxide particles were mixed together in a tissue culture flask. (Final reaction volume was 200 ml in this experiment). Mixture was allowed to mix overnight at 4°C. The particles were allowed to separate for 60 minutes by placing the tissue culture flask on a magnetic plate at room temperature. The supernatant was removed, and an aliquot was saved for estimating the amount of antibody bound to the particles. More than 98% of the antibody was bound to the particles under these conditions. The reaction was quenched with 200 ml of quench solution (2.0 M glycine buffer) and allowed to rock for one hour at room temperature. The particles were separated for 30 minutes on a magnetic plate and remove the supernatant and then washed 10 times with 150 ml of wash buffer (10 mM potassium phosphate buffer, 0.1% bovine serum albumin, pH 7.4) in a tissue culture flask. Supernatant from the last wash was discarded. 200 ml of storage buffer (10 mM potassium phosphate buffer, 0.1% bovine serum albumin, 0.01% thimerosal, pH 7.4) was added to the particle suspension and stored at 4°C. Ten µl of the particle suspension contained 10 µg of anti-CKB antibody and 250 µg of chromium dioxide.

### d) Selection of wavelengths for measuring β-galactosidase activity in an endpoint mode for CKMB.

The immunoassay for CKMB described herein for an automated immunoassay used β-galactosidase as the reporter enzyme, i.e., the detectable label. β-galactosidase activity is measured using chlorophenol red galactoside (CPRG) as substrate whereby the enzyme reacts with CPRG to produce chlorophenol red (CPR). Approximately, 3 mg of CPRG was used per assay. The maximum absorbance of CPRG was at 414 nm.

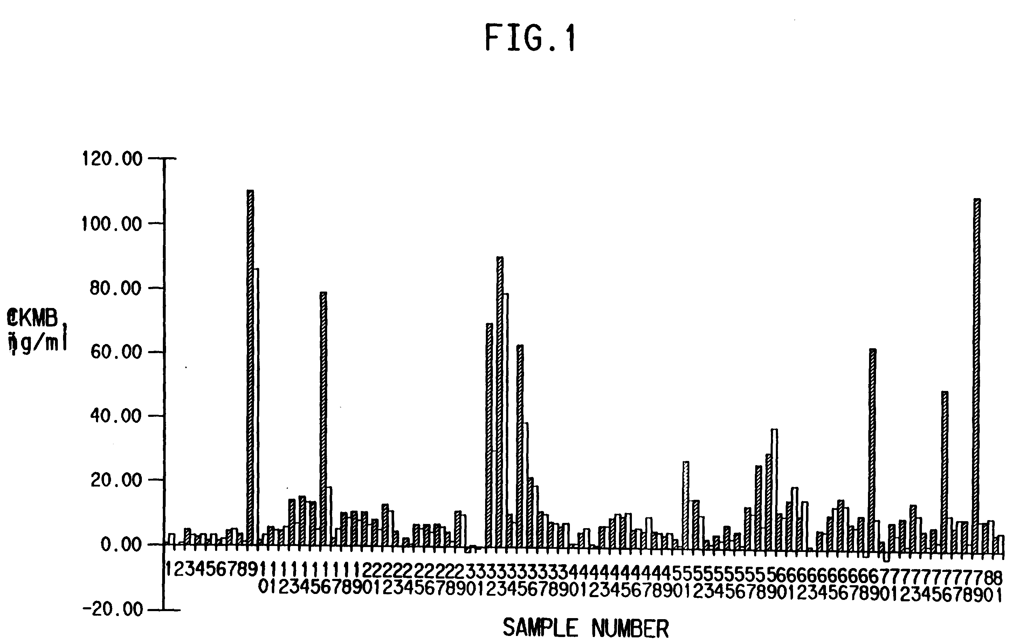

CPR, on the other hand, had a maximum absorbance at 577 nm. The amount of CPR formed by the enzyme is less than 1% of the CPRG. Figure 1 shows the absorption spectrum of chromium dioxide particles in buffer and also CPRG and CPR in the presence of chromium dioxide particles.

It was found that a bichromatic endpoint measurement gave good reproducible measurements. In using this approach one must keep in mind that the endpoint measurement also requires measurement of signal at two wavelengths. The primary wavelength selected was 577 nm because CPR absorbs maximally at that wavelength. Use of 600 nm wavelength as a blank gave satisfactory performance. It was determined that a 620 nm filter was needed to obtained best results with the detection system. Lower wavelengths such as 510 and 540 nm can also be used but they fall on the descending slope of the CPRG peak and therefore may be sensitive to variation of the CPRG concentration.

Thus, the preferred measurement system should measure the product of the β-galactosidase activity at the primary wavelength of 577 nm and secondary wavelength of 600 nm or above. Subtraction of the reading obtained from the secondary wavelength from the reading obtained from the primary wavelength allowed precise measurement of the enzyme activity.

### e) Reagents

1. CKMB Calibrators: 0, 13.8, 32, 64 and 128 ng of human CKMB per ml. Purified human CKMB (Aalto Scientific Ltd., Vista, CA) was added to CKMB-free human serum pool.
2. Anti-CKB chromium dioxide particles were prepared as described above.
3. Anti-CKMB F(ab')₂ β-galactosidase conjugate concentrate was prepared as described above.
4. Conjugate dilution buffer contained 100 g bovine serum albumin, 33.5 g, sodium PIPES, 29.2 g sodium chloride and 0.2 g magnesium chloride, per liter of deionized water, pH 7.0.
5. Wash buffer consisted of 250 mM Tris, 50 mM sodium borate, pH 7.85.
6. Resuspension buffer consisted of 0.03 M HEPES, 0.02 M sodium HEPES, 0.01 M magnesium acetate, and 0.005% Tween® 20, pH 7.6.
7. CPRG tablet containing 3 mg chlorophenol red galactoside, 8.75 mg trehalose, 30 mg mannitol and 3.15 mg carbowax® (20 µm) was prepared using the process substantially as disclosed in U.S. Patent No. 3,932,943 issued to Briggs et al., on January 20, 1976).
8. HEPES ((n-[2-hydroxyethyl]piperazine-N'-[2-ethanesulfonic acid)) tablet containing 40.4 mg sodium HEPES, 22.8 mg HEPES, 7.0 mg sorbitol, 1.15 mg magnesium acetate, and 4.0 mg carbowax (20 µm ) was prepared using the process substantially as disclosed in U.S. Patent No. 3,932,943 issued to Briggs et al., on January 20, 1976.

### f) Protocol

Ten µg of anti-CKB antibody coated on chromium dioxide particles as described above was dispensed in a reaction tube along with 300 µl of appropriately diluted conjugate into the tube. 300 µl of sample or calibrator was dispensed into the tube which was then vortexed and placed in a waterbath at 37°C for 15 min and mixed every 2 min. After the incubation period, the particles were separated by placing the tubes in a Corning magnetic test tube stand. Supernatant was aspirated and then 500 µl of wash buffer was added and the particles were resuspended. This step was repeated 3 times. 75 µl of resuspension buffer was added after the last wash step and the particles were again resuspended. 60 µl of particle suspension was dispensed into a pack.

β-galactosidase activity bound to the particles on an aca® (E. I. du Pont de Nemours and Company, Wilmington, DE 19898) was measured. aca® CPRG packs (standard aca® discrete clinical analyzer packs available from E. I. du Pont de Nemours and Company, Wilmington, DE) for use with an aca® discrete clinical analyzer, containing a HEPES tablet in dimple 2, and a CPRG tablet in dimple 3, where dimples 2 and 3 refer to tablet storage areas in a standard aca® discrete clinical analyzer pack, a CPRG tablet contains 3 mg chlorophenol red galactoside, 8.75 mg trehalose, 30 mg mannitol and 3.15 mg carbowax® [20 µm ] prepared using the process substantially as disclosed in U.S. Patent No. 3,932,943 issued to Briggs et al., on January 20, 1976),
and a HEPES tablet contains 40.4 mg sodium HEPES, 22.8 mg HEPES, 7.0 mg sorbitol, 1.15 mg magnesium acetate, and 4.0 mg carbowax® [20µm ] prepared using standard tableting processes known in the art.

The packs were then sealed with cellophane tape and the β-galactosidase activity bound to the particles was measured using the aca® discrete clinical analyzer as follows. Once the pack was loaded on the aca®, 2 ml of phosphate buffer, pH 7.8, and 3 ml of water were dispensed into the pack. Breaker mixer 1 (a component of the aca® discrete clinical analyzer which breaks tablet reagents and facilitates the mixing reagents) was utilized to dissolve the HEPES and CPRG tablets and suspend the particles. Bound enzyme reacted with CPRG to form CPR at 37°C. After 4.2 min the CPR formed was measured at 577 and 600 nm wavelengths. 577 was the primary wavelength at which the CPR has maximum absorbance, and 600 nm was the blanking wavelength. The 600 nm reading was subtracted from the 577 reading to eliminate interference due to suspended particles. The 577 minus 600 nm reading, called the endpoint reading in aca®, was plotted against the bottle values of the CKMB calibrators to generate a standard curve.

### Example 1

Eighty-one serum samples suspected to give false positive results in a CKMB immunoassay due to nonspecific binding caused by the presence of interfering antibodies were analyzed for CKMB values using the protocol described above. This assay was performed using intact anti-CKMB antibody conjugate as the detector antibody in the absence and presence of polymerized IgG.

Specifically, an anti-sulfonyl urea herbicide (Glean®, E. I. du Pont de Nemours and Company, Wilmington, DE) mouse monoclonal IgG (46/67.1.2, E. I. Du Pont de Nemours and Co., Inc., Wilmington, DE) was made using conventional techniques and was purified by protein A affinity chromatography as described above.

The antibody (46/67.1.2) solution was dialyzed against phosphate buffer containing 10 mM sodium phosphate, 300 mM sodium chloride, 0.02% sodium azide. pH 7.0. All reagents were purchased from Sigma Chemical Co., St. Louis, MO. The antibody solution was then concentrated to 4.8-5.2 mg/ml Amicon standard cell Model 8050, equipped with XM 50 membrane.

The monoclonal antibody solution at 5 mg/ml was reacted with 0.02% solution of gluteraldehyde (Sigma Chemical Co.) at 22°C for 16-24 hours. A glycine solution was then added so that the final concentration of glycine (Sigma Chemical Co.) was 0.01 M. This was to quench any residual reaction. Excess reagents such as gluteraldehyde and glycine were removed by dialysis overnight against phosphate buffer saline (Sigma Chemical Co.) at 4-8°C. The dialyzed polymerized IgG was centrifuged at 12000 rpm (Sorvall, model RC5B, E. I. du Pont de Nemours and Co., Inc., Wilmington, DE) for 15 min to remove large aggregates. The supernatant was concentrated using Amicon standard cell Model 8050, equipped with XM 50 membrane and sodium azide to a concentration of 0.1% was added. Finally, the polymerized IgG solution was filter sterilized using Nalgene disposable filterware having a 0.2 µm filter and was stored at 4°C.

Results presented in Figure 1 show that 18 of the eighty-one samples were found to have CKMB values in excess of 12 ng/ml. These values could not be reduced below 12 ng/ml which is the upper limit of normal using 24 µg of polymerized IgG. The CKMB values of these 18 samples were found to be less than 10 ng/ml using Hybritech's "Tandem®-E CKMB immunoenzymetric" assay.

### Example 2

Attempts were made to reduce the CKMB values for three samples of the eighteen mentioned above which gave the highest CKMB values by increasing the amount of polymerized IgG. Intact anti-CKMB antibody conjugate was used as the detector antibody.

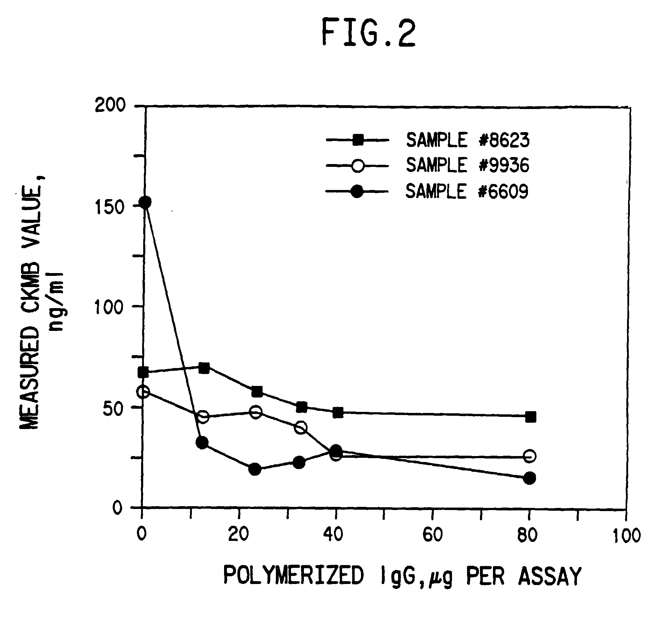

Figure 2 shows that as much as 80 µg of polymerized IgG per assay could not reduce the apparent CKMB values of these three samples below 12 ng/ml. The results suggest that polymerized IgG does not effectively reduce non-specific binding when intact antibody conjugate is used as the detector antibody in an immunoassay.

### Example 3

Two of three samples (#8623 and #6609) evaluated in Example 2 above that showed false positive results were evaluated by using various amounts of non-polymerized polyclonal mouse IgG and intact antibody conjugate.

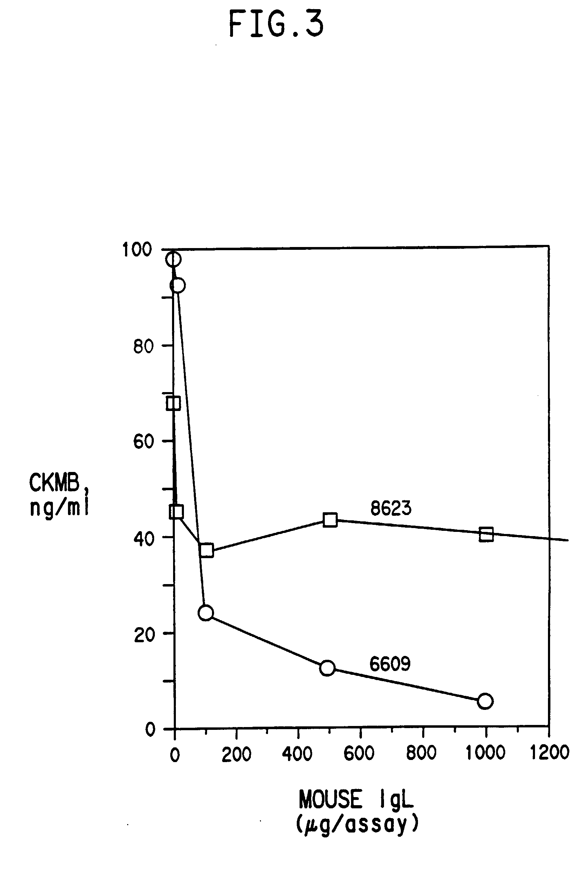

Figure 3 shows the false positive result obtained in one of the samples (sample #6609) could be reduced to a CKMB value of less than 12 ng/ml using no more than 1 ng/ml non-polymerized polyclonal mouse IgG. However, such results could not be obtained for the other sample (sample #8623), i.e., the false positive result could not be eliminated. These results suggest that non-polymerized polyclonal mouse IgG does not completely eliminate non-specific binding due to the presence of interfering antibodies in some patient samples when intact antibody conjugate is used as the detector reagent in an immunoassay.

### Example 4

Sample #6609 was then evaluated using an immunoreactive anti-CKMB antibody fragment conjugate as the detector antibody in the presence or absence of non-polymerized polyclonal mouse IgG. Specifically, F(ab')₂ coupled to β-galactosidase was used as the detector antibody. The amount of non-polymerized polyclonal mouse IgG was optimized.

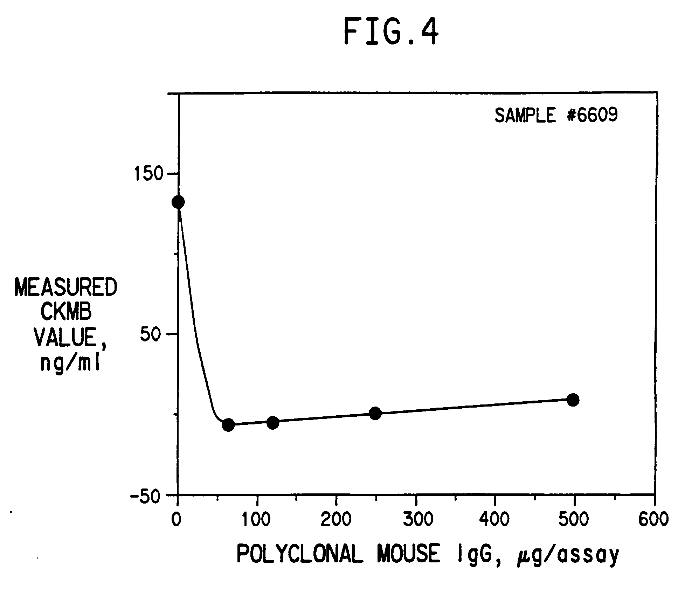

Figure 4 shows that F(ab')₂ conjugate-by itself was not effective in eliminating the false positive result in the sample. However, when the F(ab')₂ conjugate was used with as little as 50 µg of non-polymerized polyclonal mouse IgG per assay non-specific binding due to the presence of interfering antibodies in the sample was completely eliminated.

### Example 5

All 18 samples that gave false positive results as described in Example 1 above were reanalyzed using F(ab')₂ conjugate as the detector antibody in the absence and presence of polymerized IgG and polyclonal mouse IgG (Scantibodies Laboratory, Santee, CA).

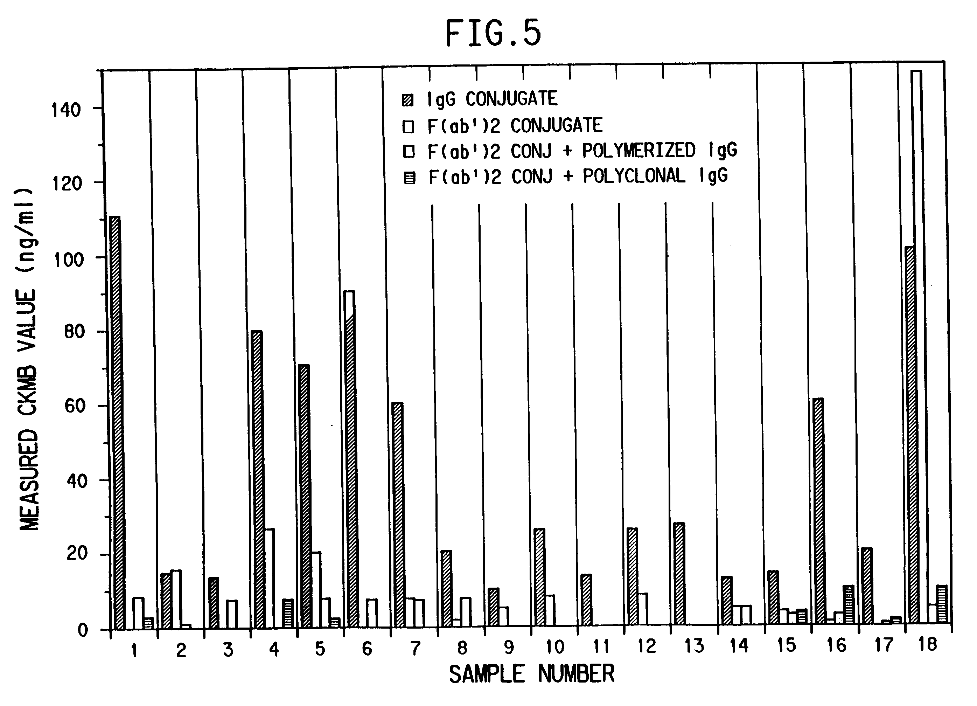

Figure 5 shows that use of F(ab')₂ conjugate and non-polymerized polyclonal IgG substantially eliminated the problem due to non-specific binding in all patient samples tested.

### Example 6

### Effect of Polyclonal Mouse IgG on Non-Specific Binding In An Immunoassay For hCG

### a) Preparation of F(ab')₂-β-galactosidase conjugate reagent

Anti-hCG antibody (Hybritech 514) used in preparing the detector antibody fragment was purchased from Hybritech Inc., P.O. Box 269006, San Diego, CA 92126. This antibody was a beta chain specific monoclonal antibody having an affinity of approximately 3 X 10⁻¹⁰ M. However, any anti-hCG monoclonal antibody having sufficient affinity for use in an immunoassay format can be used, and such antibodies can be prepared using techniques known in the art for the production of monoclonal antibodies such as those described by Kohler and Milstein, Nature, 256:495-495 (August 7, 1975). The F(ab')₂ fragment and F(ab')₂-β-galactosidase conjugate were prepared as described above in Example 1.

A solution of concentrated conjugate as prepared in Example 1 was diluted with a volume of the β-galactosidase conjugate dilution buffer (containing 100 mM PIPES, 1 mM MgCl₂, 500 mM NaCl, 10% (w/v) bovine serum albumin, and 167 µg/ml protein-A purified mouse IgG, pH 7.0) to a final concentration of 4 X 10⁻⁹ M β-galactosidase.

### b) Anti-hCG Capture Antibody Coupled to Chromium Dioxide Particles

Chromium dioxide particles were prepared substantially as described in U.S. Patent No. 4,661,408 issued to Lau et al. on April 28, 1987.

Anti-hCG antibody used as the capture antibody was obtained from Du Pont cell line 34/25. The immunogen was whole molecule hCG and the cell line was prepared using techniques known in the art for the production of monoclonal antibodies. The antibody used was an alpha-chain specific anti-hCG monoclonal antibody having an affinity of approximately 3 x 10⁻¹⁰ M. However, any anti-hCG monoclonal antibody having sufficient affinity for use in an immunoassay format can be used, and such antibodies can be prepared using techniques known in-the art for the production of monoclonal antibodies such as the method of Kohler and Milstein noted above.

Capture anti-hCG antibody was coupled to chromium dioxide particles as described above in Example 1. To a slurry of chromium dioxide particles containing 25 mg/ml solids was added anti-hCG capture antibody at a final concentration of 0.75 mg/ml. The anti-HCG capture antibody coated chromium dioxide particle slurry thus obtained was made into tablets containing 3.0 mg antibody-coated chromium dioxide particles per tablet, with 9.83 mg trehalose, 0.98 mg carbowax®, and 0.03 mg trimerosal added as inert materials. The tablets were prepared using the process substantially as disclosed in U.S. Patent No. 3,932,943 issued to Briggs et al., on January 20, 1976.

### c) Effect of Polyclonal IgG on Non-Specific Binding In an Immunoassay for hCG

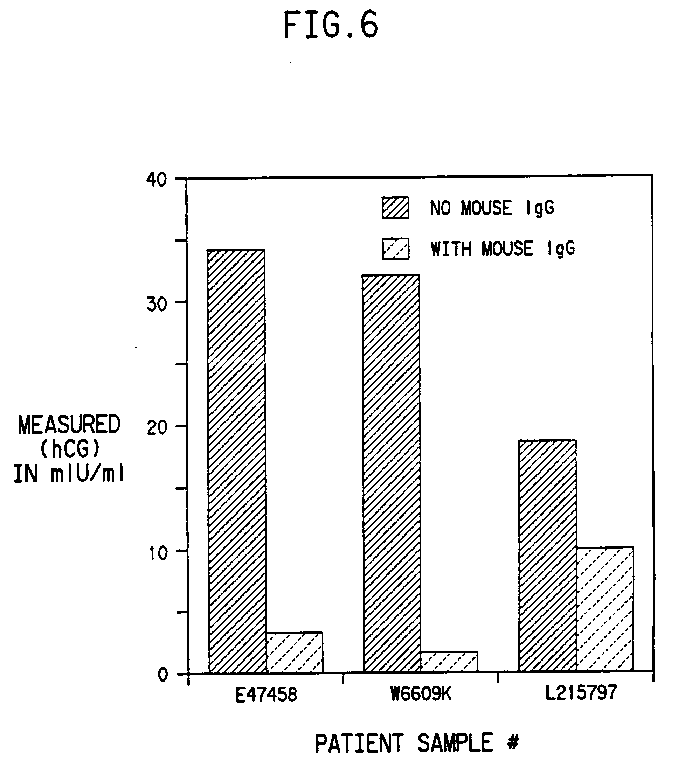

Five tablets containing anti-HCG capture antibody coated chromium dioxide particles were dissolved in 3 ml water and the tubes were incubated for 15 minutes at room temperature. 75 µl of above-described suspension containing 250 µg of chromium dioxide particles solids was dispensed into 22, 12 mm x 75 mm polyethylene tubes. 100 µl of each of a series of hCG calibrators (0, 25, 100, 300 and 500 mIU/ml of human hCG per ml; hCG was purified from the urine of pregnant women and standardized according to the 1^{st} International Reference Preparation [First IRP] from the World Health Organization) was added to 10 tubes (duplicate tubes were prepared for each calibrator concentration), the contents of which were then mixed by vortexing and placed in a 37°C heating block for 2 minutes. Similarly, 100 µl of each of three patient samples was added to 12 tubes (two sets of duplicate tubes were prepared for each patient sample), the contents of which were then mixed by vortexing and placed in a 37°C heating block for 2 minutes. 400 µl of appropriately diluted F(ab')₂-β-galactosidase conjugate reagent (4x10⁻⁹ M β-galactosidase-tag antibody conjugate diluted with conjugate dilution buffer containing 100 mM PIPES, 1 mM MgCl₂, 500 mM NaCl, 10% (w/v) bovine serum albumin, and 167 µg/ml protein-A purified mouse IgG, pH 7.0) was added into each of the 10 tubes containing the calibrators and into six of the tubes containing patient samples (two tubes for each patient sample); the contents of each tube were mixed by vortexing and then incubated at 37°C for 15 minutes. Similarly, 400 µl of appropriately diluted F(ab')₂ β-galactosidase conjugate reagent (β-galactosidase-tag antibody conjugate diluted with conjugate dilution buffer containing 100 mM PIPES, 1 mM MgCl₂, 500 mM NaCl, 10% (w/v) bovine serum albumin, and no polyclonal mouse IgG) was added into each of the remaining six tubes containing patient samples (two tubes for each patient sample); the contents of each tube were mixed by vortexing and then incubated at 37°C for 15 minutes. After 15 minutes, the particles contained in all of the tubes were separated by placing the tubes in a magnetic test tube stand such that the particles were held magnetically against the sides of the test tube. The supernatant of each tube was removed by aspiration, 500 µl of wash buffer (250 mM Tris, 50 mM sodium borate, pH 7.85) was added to each tube and the particles were resuspended by vortexing. The wash procedure was repeated two additional times. After the last wash, 75 µl of resuspension buffer (0.03 M HEPES, 0.02 sodium HEPES, 0.01 M magnesium acetate, and 0.005% Tween^{R}20, pH 7.6) was added, the particles were resuspended by vortexing, and 60 µl of particles were dispensed into dimple 1 of aca® CPRG packs standard aca® discrete clinical analyzer packs (available from E. I. du Pont de Nemours and Company, Wilmington, DE for use with the aca® discrete clinical analyzer, containing a HEPES tablet in dimple 2, and a CPRG tablet in dimple 3, where dimples 2 and 3 refer to tablet storage areas in a standard aca® discrete clinical analyzer pack, a CPRG tablet contains 3 mg chlorophenol red galactoside, 8.75 mg trehalose, 30 mg mannitol and 3.15 mg carbowax® [20 µm ] prepared using the process substantially as disclosed in U.S. Patent No. 3,932,943 issued to Briggs et al., on January 20, 1976), and a HEPES tablet contains 40.4 mg sodium HEPES, 22.8 mg HEPES, 7.0 mg sorbitol, 1.15 mg magnesium acetate, and 4.0 mg carbowax® [20 µm ] prepared using standard tableting processes known in the art. The packs were sealed with cellophane tape and the activity of β-galactosidase bound to the particles measured on the aca®. Measurements were obtained on an aca® as follows. Once the pack was loaded on the aca®, 2 mL of phosphate buffer, pH 7.8, and 3 ml of water was dispensed into the pack. Breaker mixer 1 (a component of the aca® discrete clinical analyzer which breaks tablet reagents and facilitates the mixing of reagents) was utilized to dissolve the HEPES and CPRG tablets and suspend the particles. Bound β-galactosidase enzyme reacted with CPRG to form chlorophenol red (CPR) at 37°C. After 4.2 minutes the CPR formed was measured at 577 and 600 nm wavelengths. 577 is the primary wavelength at which the CPR has maximum absorbance, and 600 nm is the blanking wavelength. The 600 nm reading was subtracted from the 577 reading. The measurement at 577 nm minus the measurement at 600 nm, called the bichromatic endpoint reading on the aca®, was plotted against the concentration of the hCG calibrators to generate a standard curve. The results are presented in Figure 6. Figure 6 shows the hCG measurements determined for each of the three patient samples in the presence and absence of non-polymerized mouse IgG. The results indicate the effect of non-polymerized mouse IgG in reducing false positive results in an immunoassay for hCG.

## Claims

1. A method for substantially eliminating erroneous results in an immunoassay due to the presence of interfering antibodies which comprises reacting an immunoreactive antibody fragment conjugate as the detector antibody and non-polymerized IgG with the interfering antibodies present in a sample to be tested for the presence or absence of an analyte.

2. A method according to claim 1 wherein the immunoreactive fragment is selected from the group consisting of Fab, Fab', or F(ab')₂.

3. A method according to claim 1 wherein the species is selected from the group consisting of mouse, rat, rabbit, goat or horse.

4. A method according to claim 1 wherein the antigen is selected from the group consisting of CKMB, TSH, or hCG.

5. A method according to claim 1 wherein the non-polymerized IgG is from the same species as the capture antibody and the conjugate.

## Patentansprüche

1. Verfahren zur weitgehenden Beseitigung durch die Gegenwart störender Antikörper verursachter fehlerhafter Ergebnisse bei einem Immunoassay, umfassend das Umsetzen eines Konjugats eines immunoreaktiven Antikörperfragments als Detektorantikörper und von nichtpolymerisiertem IgG mit den störenden Antikörpern, die in einer auf Anwesenheit oder Abwesenheit eines Analyten zu testenden Probe vorhanden sind.

2. Verfahren gemäß Anspruch 1, wobei das immunoreaktive Fragment aus der Gruppe ausgewählt ist, die aus Fab, Fab' und F(ab')₂ besteht.

3. Verfahren gemäß Anspruch 1, wobei die Spezies aus der Gruppe ausgewählt ist, die aus Maus, Ratte, Kaninchen, Ziege und Pferd besteht.

4. Verfahren gemäß Anspruch 1, wobei das Antigen aus der Gruppe ausgewählt ist, die aus CKMB, TSH oder hCG besteht.

5. Verfahren gemäß Anspruch 1, wobei das nichtpolymerisierte IgG von derselben Spezies stammt wie der Abfang-Antikörper und das Konjugat.

## Revendications

1. Un procédé d'élimination sensiblement complète, dans un test immunologique, des résultats erronés dus à la présence d'anticorps d'interférence, qui consiste à faire réagir un conjugué d'un fragment d'anticorps immunoréactif comme anticorps détecteur et une IgG non-polymérisée avec les anticorps d'interférence dans un échantillon à tester pour détecter la présence ou l'absence d'un analyte.

2. Un procédé selon la revendication 1 dans lequel le fragment immunoréactif est choisi dans le groupe constitué par : Fab, Fab', ou F(ab')₂.

3. Un procédé selon la revendication 1 dans lequel l'espèce est choisie dans le groupe constitué par: souris, rat, lapin, chèvre ou cheval.

4. Un procédé selon la revendication 1 dans lequel l'antigène est choisi dans le groupe constitué par: CKMB, TSH, ou hCG.

5. Un procédé selon la revendication 1 dans lequel l'IgG non polymérisée est de la même espèce que l'anticorps de capture et le conjugué.
